Europäisches Patentamt

European Patent Office

Office européen des brevets

(11) Publication number: **0 213 241**
**A1**

# (12) EUROPEAN PATENT APPLICATION

(21) Application number: 85305700.8

(22) Date of filing: 12.08.85

(51) Int. Cl.⁴ **A61F 13/20**

(43) Date of publication of application:
**11.03.87 Bulletin 87/11**

(84) Designated Contracting States:
**DE FR GB**

(71) Applicant: **CREATIVE RESOURCES, INC.**
**34 Cascade Road**
**Stamford Connecticut 06903(US)**

(72) Inventor: **Fuller, William B.**
**34 Cascade Road**
**Stamford Connecticut 06903(US)**

(74) Representative: **Brooke-Smith, Fred et al**
**STEVENS, HEWLETT & PERKINS 5 Quality**
**Court Chancery Lane**
**London WC2A 1HZ(GB)**

(54) **System for packaging, handling and disposing of consumer products.**

(57) A packaging system including a receptacle (30) for sanitary disposal of a spent article(58) according to which the same receptacle(30) also serves to protectively envelop a replacement article. The system also enables the sanitary removal and handling of a spent article(58) according to which the same receptacle also serves to protectively envelop a replacement article. The receptacle, composed of a relatively thin, pliable, liquid impervious material, is generally tubular shaped, sealed at one end and open at the other end. It can be flattened to define an upper ply and a lower ply, then wrapped about a replacement article placed on the upper ply to protectively envelop the replacement article. The wrapped condition is maintained until the replacement article is needed at which time the receptable is unwrapped to expose the replacement article. A user's fingers or hand(56) can be extended into the receptacle to grasp and hold a spent article without touching it. The receptacle is then drawn inside out to envelop the spent article and the opening sealed enabling the sanitary disposal of the receptacle and spent article therein.

FIG. 10.

## SYSTEM FOR PACKAGING, HANDLING AND DISPOSING OF CONSUMER PRODUCTS

This invention relates to a novel system of packaging according to which a receptacle for a new or replacement article is also used for the sanitary handling and eventual disposal of a spent article.

The sanitary handling and, particularly, disposal of personal hygiene products such as sanitary napkins remains a problem in our present society. To date, there is no known "sanitary" means of disposal. Disposable diapers pose a similar problem when spent away from home and running water. Many other consumer goods likewise fall into this category of items seeking a solution to the problem of disposal, when spent, in a sanitary and tasteful manner. Throughout this description, the term "replacement article" merely refers to any new or fresh item which is to be sold to a consumer for future use. The type of item with which the receptacle is to be used is not considered to be a limitation of the invention since the receptacle is useable with an almost limitless number of items, whether manufactured or found in nature. Similarly, the term "spent article" merely refers to any old, used, broken, or soiled item which is no longer considered to have usefulness such that disposal is properly sought for the item.

The prior art has recognised this problem and, in some instances, has provided solutions to certain aspects of the problem. For example, the patent to Peterkin, No. 1,725,996, issued August 27, 1929, discloses a plurality of tampons in individual sealed envelopes. Each envelope can be opened to provide a tampon as desired. However, the patent does not mention disposal of a spent tampon. Each of the patents to Crockford, No. 3,058,469, issued October 16, 1962, and to Sneider, No. 4,351,339, issued September 28, 1982, discloses a tampon and an applicator for its sanitary insertion; and each also discloses structure for the removal of the tampon when spent. However, there is no mention in either patent of the disposal of a spent tampon. The patent to Pirie, No. 3,298,369, issued January 17, 1967, similar to the Crockford and Sneider patents, discloses a tampon, an applicator for its sanitary insertion, and structure for removal of a spent tampon. In this instance, however, there is mention of disposal of a spent tampon, namely, by dropping it and its contents in a toilet bowl simultaneously with removal (Col. 3, Ins. 17-19 and Col. 4, Ins. 63-65).

The patent to Collett et al., No. 3,103,930, issued September 17, 1963, discloses a specialised diaper or container for receiving body wastes of infants, incapables, and hospitalised persons. The container includes an appropriate opening to receive the wastes and the opening can be closed to retain the waste material when it is desired to dispose of the container.

Perhaps the most pertinent patents known to the inventor are those, respectively, to McMahan, No. 2,478,412, issued August 9, 1949, to Pickens, No. 2,750,033, issued June 12, 1956, and to Elmore, No. 3,035,578, issued May 22, 1962.

A packaging arrangement is disclosed in McMahan according to which a plurality of sanitary napkins are contained in a box together with a like number of envelopes for their eventual disposal. It is contemplated that each of the items be withdrawn as it is needed. The patent to Pickens discloses an elongated paper envelope for protecting a new sanitary napkin and for subsequently receiving the used napkin for its disposal. The envelope can be opened to expose the new napkin and includes closure means for its resealing after it has received a used napkin. In Elmore, a device is disclosed for enclosing a used sanitary napkin. A disposable cover is removably attached to the sanitary napkin. After the napkin has been used, it is wrapped within the cover for disposal. In another embodiment of the invention, a bag similarly used to receive and dispose of a used napkin is retained in a pocket on the underside of the napkin until needed.

While each of the aforesaid patents, on its face, discloses advances in the state of the art of sanitary devices existing when each respective patent was granted, nonetheless, the present invention is deemed to be a considerable improvement over such known devices. Indeed, it was with recognition of a need and of the state of the prior art that the present invention was conceived and has now been reduced to practice.

In accordance with the invention, then, a packaging system is disclosed according to which a protective outer wrap member for a new article also provides for contamination free handling of a spent article and serves as a receptacle for disposal of the spent article comprising: a generally tubular shaped receptacle sealed at one end and having an opening at the other end defined by a rim movable between open and closed positions, said receptacle composed of relatively thin, pliable, liquid impervious material, said receptacle having a first surface defining a cavity extending between said sealed end and said rim and a second surface being the outer surface of said receptacle, said receptacle capable of being flattened to define a lower ply and an upper ply congruent with said lower ply, said receptacle further capable of being wrapped about and protectively enveloping a replacement article placed on said upper ply; first closure means for selectively maintaining the wrapped condition of said receptacle about the replacment article, said closure means being selectively releasable and said receptacle capable of being selectively unwrapped to expose the replacement article, whereupon, following removal from the cavity of the replacement article, at least a part of a user's hand is receivable through said rim and within the cavity and is able to grasp and hold a spent article without touching the spent article; and second closure means adjacent said rim for holding said rim closed after said receptacle has been turned inside out and the spent article enveloped by said second surface, said receptacle and the spent article contained therein thereby being in condition for sanitary disposal.

A novel packaging system is disclosed which includes a receptacle for sanitary disposal of a spent article according to which the same receptacle also serves to protectively envelop a replacement article. The system also enables the sanitary removal and handling of a spent article by the user as it is placed in the receptacle for sanitary disposal. The receptacle, composed of a relatively thin, pliable, liquid impervious material, is generally tubular shaped, sealed at one end and open at the other end. It is flattened to define an upper ply and a lower ply, then wrapped about a replacement article placed on the upper ply to protectively envelop the replacement article. The wrapped condition is maintained until the replacement article is needed at which time the receptacle is unwrapped to expose the replacement article. A user's fingers or hand can be extended into the receptacle to grasp and hold a spent article without touching it. The receptacle is then drawn inside out to envelop the spent article and the opening sealed enabling the sanitary disposal of the receptacle and spent article therein.

The present invention, then, is directed to a novel packaging system used to provide for the sanitary handling, shipping, storage, and disposal of a variety of products, especially those products which relate to personal hygiene. The same receptacle which serves as the protective outer wrap or covering for a new or replacement article also serves to receive the spent article being replaced. This not only provides substantial convenience to the user, but may also provide the economy which results when the outer wrapper and disposal envelope are one and the same device. Thus, instead of the outer wrapper being discarded and not used further when a user seeks out a replacement article, its usefulness continues in that it permits the user to remove the spent article without contaminating her hands and, following such removal, to provide still a further benefit of receiving and enveloping the spent article for its sanitary disposal. The products with which the invention may be used include tampons, sanitary napkins, disposable diapers, medical products such as bandages, nonconsumable chewing products such as gum and tobacco, tea bags, and a host of other items.

In accordance with the invention, individual products are actually shipped to the end user wrapped in the sanitary disposal unit itself. The consumer unwraps the desired product and uses the unwrapped package for handling of a spent product to be replaced and, subsequently, disposing of the spent product in a completely sanitary manner. Thus, opening the new product provides the sanitary means for disposal of the old or spent product.

A primary feature of the invention is the provision of a single device which serves, variously, as an exterior package wrap providing sanitary protection of an enclosed article, as a device which enables a user to handle spent articles in a nonsanitary environment without becoming contaminated, and as a suitable disposable bag for receiving and enclosing, in a sanitary fashion, a spent article. Another feature of the invention is the provision of an outer wrap for an absorbent item which provides protection from dirt and moisture to maintain the freshness of that item. The same outer wrap also enables handling of a spent item while maintaining the cleanliness and dryness of the handling party; and for keeping moisture and waste products contained in the spent item from penetrating the disposal bag. Another feature of the invention resides in the ability it offers of being available for use whenever and wherever it is needed without the need of access to water or other cleaning facilities. Yet a further benefit of the invention is the means it offers of disposing of a spent article in a discreet and tasteful manner.

The invention will now be described in more detail. The description makes reference to the accompanying diagrammatic drawings in which:

Figure 1 is a perspective view of a receptacle embodying the principles of the invention;

Figure 2 is a perspective view illustrating one step of a procedure using the receptacle of Figure 1;

Figure 3 is a detail cross section view taken generally along line 3--3 in Figure 2;

Figure 4 is a side elevation view of a package created from the procedure illustrated in Figure 2, certain parts being cut away and in section;

Figure 5 is a cross section view taken generally along line 5--5 in Figure 4;

Figure 6 is a detail perspective view, similar to Figure 2 illustrating another embodiment of the invention;

Figure 7 is a perspective view of a completed package embodying the principles of the present invention;

Figure 8 is a perspective view, similar to Figure 7, illustrating another embodiment of the invention;

Figures 9, 10 and 11 are successive perspective views illustrating successive steps in a procedure performed according to the teachings of the invention;

Figures 12, 13, and 14 are detail side elevation views illustrating various closures utilised with the invention;

Figure 15 is a detail cross section view taken generally along line 15--15 in Figure 2;

Figure 16 is a top elevation view of another embodiment of the receptacle illustrated in Figure 1;

Figure 17 is detail side elevation view, similar to Figures 12, 13, and 14 illustrating another type of closure for the invention;

Figure 18 is a perspective view, certain parts cut away and in section, illustrating another manner of use of the invention;

Figures 19 and 20 are detail side elevation views similar to Figures 12-14, and 17, illustrating preferred embodiments of the closure for a receptacle; and

Figure 21 is a cross section view taken generally along line 21--21 in Figure 20.

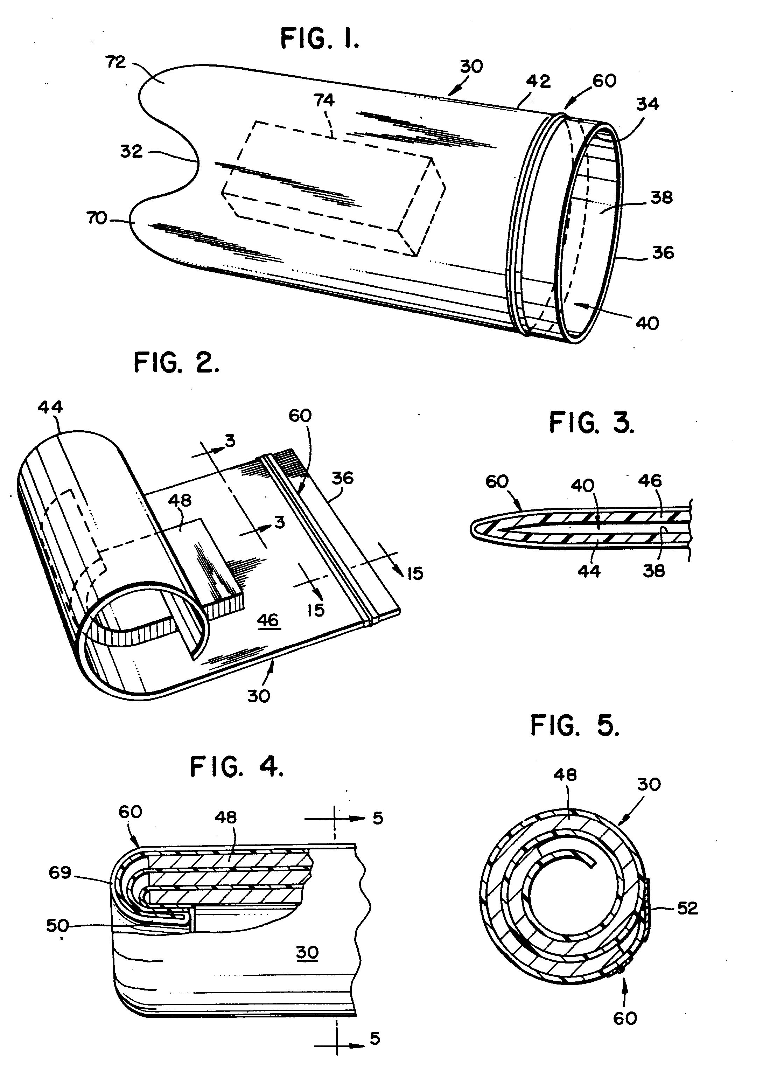

Refer now to the drawings, and initially to Figures 1, 2, and 3 which generally illustrate a receptacle 30 which embodies the principles of the present invention.

As embodied herein, and with continuing reference to Figures 1-3, it will be seen that the receptacle 30 is generally tubular shaped, sealed at an end 32 and having an opening 34 at its end opposite the sealed end 32. The opening 34 is defined by a rim 36 which is movable between open and closed positions (see Figures 1, and 3, respectively). The receptacle 30 may be composed of any suitable material, polyethylene being one example, but, preferably, it should be relatively thin, pliable, and impervious to liquids. The receptacle 30 has a first surface 38 which defines a cavity 40 which extends between the sealed end 32 and the rim 36. The receptacle 30 is also defined by a second surface 42 which is the outer surface when the receptacle is in the condition illustrated in Figure 1.

As seen in Figures 2 and 3, the receptacle 30 is capable of being flattened to define a lower ply 44 and an upper ply 46 which is congruent with the lower ply. In actual fact, the receptacle 30 may be formed from one continuous piece of material. That is, the term "ply" should not be taken to mean necessarily that the receptacle 30 is formed from two pieces of material; rather, the term "ply" is intended to mean a separate portion of the receptacle as defined by the terms "upper ply" and "lower ply".

Turning to Figures 2, 4, and 5, it is seen that the receptacle 30 is capable of being wrapped about and protectively enveloping a replacement article 48 placed on the upper ply 46. The particular replacement article 48 illustrated in the Figures 2, 4, and 5 may be, for example, a sanitary napkin which is generally flat and rectangular in shape. It is preferable that the lateral and longitudinal dimensions of the receptacle 30 be greater than those of the replacement article 48 when article 48 is readied for packaging. By so doing, when the receptacle 30 has been fully rolled as illustrated in Figure 5, the opposed edge portions of the receptacle can be tucked in as illustrated at 50 in the Figure 4 to assure that the receptacle fully envelops the replacement article 48.

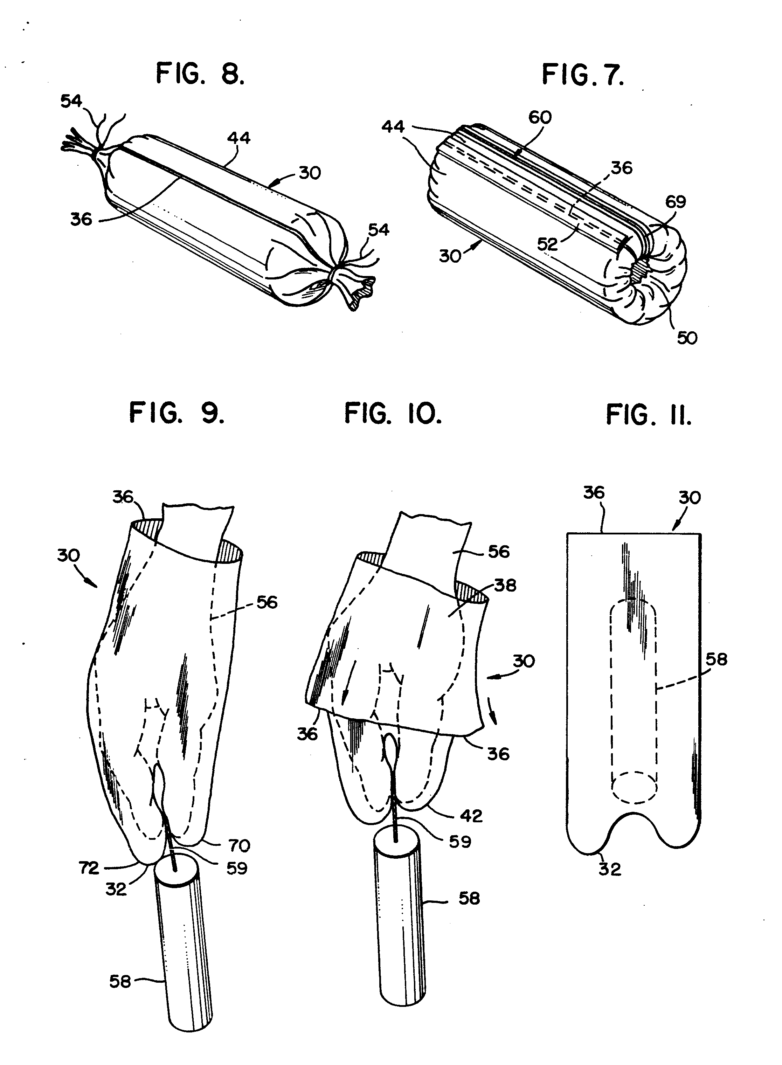

While the example of the replacement article 48 illustrated in Figure 2 is that of a generally flat, rectangular shape, the receptacle can be used to wrap articles having a variety of other shapes. As an example, a replacement article 48A is illustrated in Figure 6 as being cylindrical in shape. In actual fact, it may be in the nature of a tampon.

Closure means for selectively maintaining the wrapped condition of the receptacle 30 containing the replacement article are illustrated in Figures 7 and 8. In the Figure 7 embodiment, with the edges of the receptacle tucked in as indicated at 50, the wrapped condition of the receptacle 30 can be substantially maintained. A further aid in maintaining the wrapped condition of the receptacle is indicated by a strip of pressure sensitive tape 52 which is drawn across the rim 36 to spaced apart portions of the lower ply 44. Of course, it will be understood that various other suitable means not disclosed herein may be employed for this purpose and the invention is not restricted to merely those instrumentalities which are disclosed.

In Figure 8, instead of the edges of the receptacle 30 being tucked in as illustrated in Figures 4 and 7, they are drawn together at either end of the resulting package and tie wraps 54 are twisted together in a known fashion to properly seal the package. The configuration illustrated in Figure 8 may be more suitable for maintaining the wrapped condition of a cylindrical item such as the replacement article 48A.

Thus, packages generally having the configuration illustrated in Figures 7 and 8 are of the form in which they would be made available to the consumer. Appropriate portions of the lower ply 44 can be provided in a suitable fashion with a label and advertising material, and a number of packages may be received in a larger container for bulk shipment and for quantity purchases.

When a consumer has need of a replacement article, she merely takes a package and unwraps it, following a reverse procedure to that already described in assembling the package. She then opens the package, unrolls the receptacle 30, removes the replacement article, and inserts her fingers or hand through the rim 36 and into the cavity 40, until the digits, that is, the fingers and thumb of her hand, reach the sealed end 32. See Figure 9. The digits of the user's hand so received in the receptacle 30 can grasp and hold a spent article and remove it from its place of use, all without touching the spent article. At the same time, because of the thin walled construction of the receptacle, the user can firmly grasp and hold the spent article. Also, it is within the scope of the invention to provide a specially prepared surface of the receptacle in the region of the end 32, or to provide other suitable means, to enhance the ability of the user to obtain a firm grip on the article.

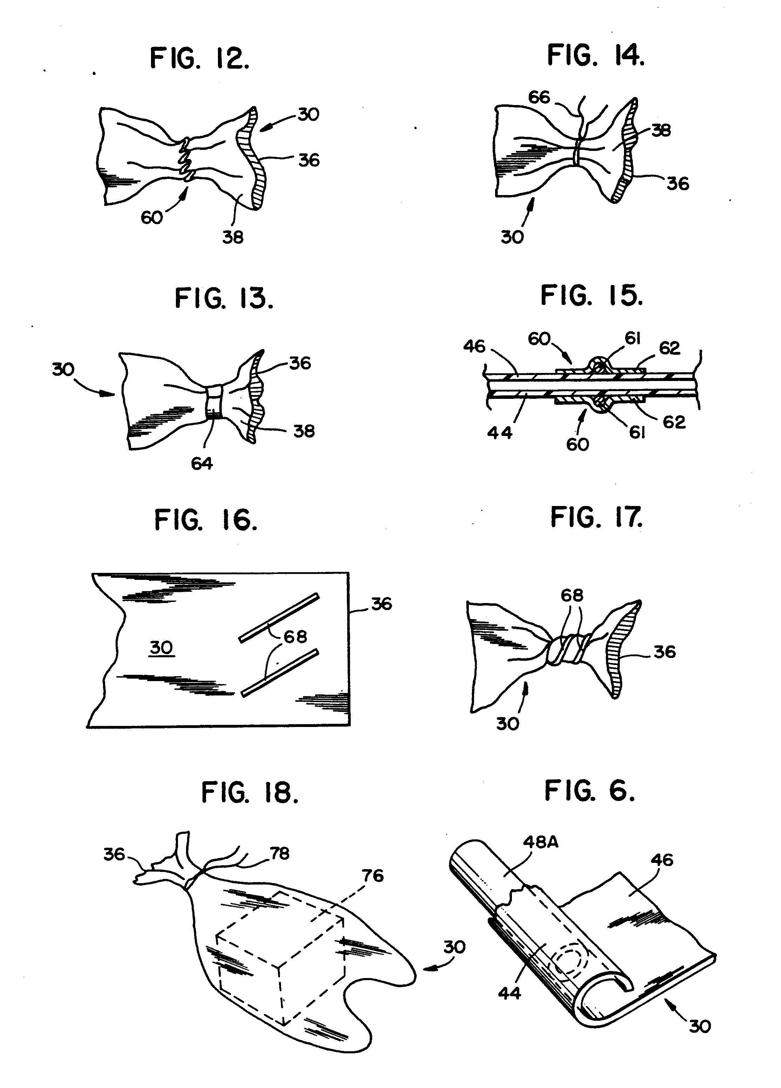

In the event the spent article is a tampon, as indicated at 58, it would be necessary to grasp a tethering string 59 (see Figures 9 and 10) customarily used for removal from its place of use. In that instance, after grasping the tethering string 59 and removing the tampon 58, the user then takes hold of the rim 36 with her other hand (see Figure 10) and draws it across the second surface 42, then over and about the spent tampon 58, in effect, turning the receptacle 30 inside out. In the final stage of this procedure, as illustrated in Figure 11, the spent tampon 58 is received totally within the receptacle surrounded by the second surface 42, now an interior surface, and formerly the outer surface of the receptacle. After the spent article is fully contained within the receptacle 30, as just described, closure means adjacent the rim 36 are used for holding the rim closed, various closures being illustrated in Figures 12, 13, 14 17, 19, and 20.

In accordance with the invention, a packaging system is disclosed which is generally as previously described wherein said second closure means includes a tie wrap mounted to said receptacle generally coextensive with and adjacent to said rim, said tie wrap adapted to be twisted about itself when said rim is drawn to its substantially closed position to seal said receptacle after receiving therein the spent article. As embodied herein with particular reference to Figures 1-4, 7, and 12, a tie wrap 60 is mounted to the receptacle 30 generally coextensive with and equidistant from, the rim 36. The tie wrap is illustrated as being continuous and is preferably so, but it may also be discontinuous in its construction. The tie wrap 60 is composed of a length of metallic or plastic wire which is malleable, that is, capable of retaining whatever configuration is imposed on it. Appropriate tape 62 which may be pressure sensitive or secured in place in any other suitable fashion as by heat sealing, overlies the wire 61 covering it for its entire length as it encircles the receptacle 30. The tape 62 itself may be plastic, cloth, a rubberised fabric or any other suitable material which will not interfere with the characteristics of the malleable wire 61. Hence, as illustrated in Figure 12, the tie wrap 60 can be twisted about itself when the rim is drawn to its substantially closed position to thereby seal the receptacle which already contains the spent article 58.

In accordance with the invention, a packaging system is generally as previously described wherein said second closure means includes pressure sensitive tape fixedly applied to said first surface at locations proximate to and generally equidistant from said rim when said rim is drawn to its substantially closed position to hold said rim in its closed position. As embodied herein, with reference to Figure 13, pressure sensitive tape 64 is applied to and around the surface 38 at locations proximate to and generally equidistant from the rim 36 to hold the rim in its closed position. In a similar fashion, as illustrated in Figure 14, an independent tie wrap 66 may be received around the first surface 38, drawn tightly against the surface, then twisted to seal the receptacle 30 adjacent the rim 36.

In accordance with the invention, a packaging system is disclosed and is generally as previously described wherein said second closure means includes a pair of tie wraps mounted to said receptacle at spaced apart locations adjacent to said rim, said tie wraps adapted to be twisted about one another when said receptacle is drawn to its substantially closed position to seal said receptacle after receiving therein the spent article. As embodied herein, with particular reference to Figures 16 and 17, a pair of tie wraps 68 of a finite length but otherwise similar in construction to tie wrap 60 are mounted to the receptacle 30 at spaced apart locations adjacent to the rim 36. The tie wraps 68 may be parallel to one another, but that is not critical to their operation. When the spent article 58 is received within the receptacle 30 and the receptacle is drawn to a closed condition adjacent to the rim 36, the tie wraps 68 can be twisted about one another in the manner illustrated in Figure 17 to thereby seal the receptacle.

In accordance with the invention, a packaging system is disclosed which is generally as previously described wherein said first and second closure means are the same structure. As embodied herein, with particular reference to Figures 1, 2, 4, and 7, it is seen that the tie wrap 60 can be used for both sealing the package in which the receptacle 30 protectively envelops the replacement article 48 and, subsequently, when the receptacle receives the spent article 58 for disposal. As particularly well seen in Figures 4 and 7, the tie wrap 60 is bent over and in, as at 69, where the edges of the receptacle are tucked in at either end of the package. The solid structure of the wire 61 within the tie wrap assures against unwarranted unwrapping of the package. At the same time, the tie wrap can be readily returned to its original condition by the user to enable the receptacle 30 to be unwrapped from the replacement article 48. Subsequently, when it becomes time for the receptacle 30 to receive the spent article 58, the tie wrap 60 can be twisted about itself as previously described and as illustrated in Figure 12. In this manner, the closure means is an integral element of the receptacle 30 and need not be separately applied by the user when it comes time to dispose of the spent article 58.

In accordance with the invention, a packaging system is disclosed generally as previously described wherein said sealed end of said receptacle includes at least two spaced apart finger elements, each adapted to receive therein at least one digit of the user's hand. As embodied herein, with particular reference to Figures 1 and 9, the end 32 of the receptacle 30 is formed with at least a pair of spaced apart finger elements 70 and 72, respectively. The finger elements are adapted to receive, for example, the thumb and forefinger, respectively, of the user's hand as an aid in assuring a firm grip on the spent article itself, or possibly on the tethering string 59 (Figure 9) often provided with an article for its removal when spent. It is within the scope of the invention for the end 32 to have additional finger elements should that be desired.

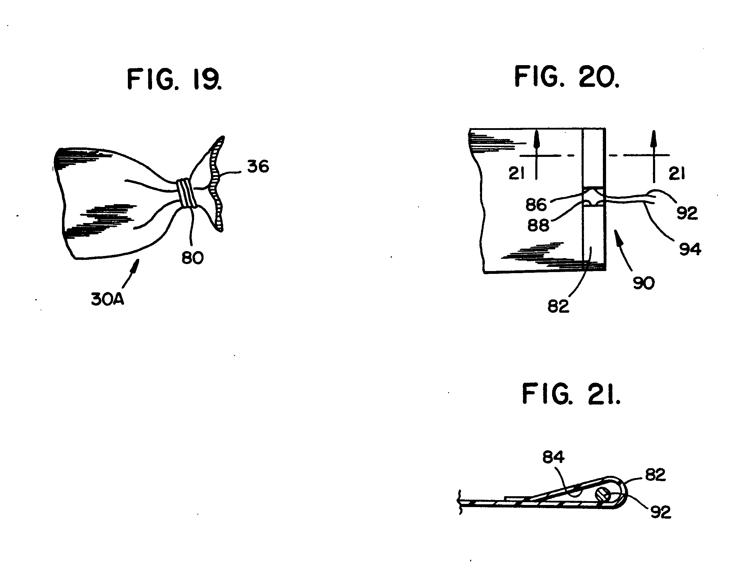

While the disclosure above has discussed the original wrapping for a replacement article 48 or 48A being in a contiguous relationship with the upper ply 46, it is considered to be within the scope of the invention for the receptacle to receive a replacement article 74 (see Figure 1) within the cavity 40. The article 74 is illustrated as being relatively flat, such as a diaper. However, a replacement article 76 (see Figure 18) might be of a bulky nature. In the latter instance, a tie wrap 78 is illustrated as a desirable manner of sealing the receptacle but it will be understood that other suitable means of closure might be acceptable. Examples of preferred constructions for sealing the receptacle are illustrated in Figures 19 and 20.

In the instance of the construction illustrated in Figure 19, closure means is in the form of a suitable elastic band 80 which is integral with a modified receptacle 30A or mounted in any suitable fashion so that it extends continuously around the receptacle adjacent the rim 36. The band 80 is chosen so that it has sufficient elasticity to draw and hold the end of the receptacle adjacent said rim in the closed position. At the same time, the elastic band is capable of being stretched, manually or otherwise, to permit the rim 36 to be moved to the open position.

In the embodiment of Figure 20, a modified receptacle indicated by reference numeral 30B includes a hem 82 formed by turning the material of the receptacle back onto itself in a continuous fashion around the cirumferential rim, then suitably bonding the former rim onto the outer surface of the receptacle to form a tube 84 having opposed openings 86 and 88. A drawstring 90 of finite length extends through the hem and includes ends 92 and 94 which extend beyond the openings 86 and 88, respectively. The ends 92 and 94 can be manipulated in a customary fashon to draw the hem to the closed position. The ends 92 and 94 can then be suitably tied to maintain the hem in the closed position. Subsequently, when desired, the ends 92 and 94 can be untied and the drawstring 90 relaxed to permit the hem 82 to be moved to the open position.

The closure means disclosed in Figures 19 and 20 can thus be used both for closure of the original wrapping for a replacement article and, subsequently, for closure of the receptacle when it receives a spent article.

While the preferred embodiments of the invention have been disclosed in detail, it should be understood by those skilled in the art that various modifications may be made to the illustrated embodiments without departing from the spirit and the scope thereof as described in the specification and defined in the appended claims.

## Claims

1. A package system according to which a protective outer wrap member for a new article also provides for contamination free handling of a spent article and serves as a receptacle for disposal of the spent article comprising: a generally tubular receptacle sealed at one end and having an opening at the other end defined by a rim movable between open and closed positions, said opening being of a size to accept a human hand, said receptacle composed of relatively thin, pliable, liquid impervious material, said receptacle having a first surface defining a cavity extending between said sealed end and said rim and a second surface being the outer surface of said receptacle, said receptacle capable of being flattened to define a lower ply and an upper ply congruent with said lower ply, said receptacle further capable of being wrapped about and protectively enveloping a replacement article placed on said upper ply; first closure means for selectively maintaining the wrapped condition of said receptacle about the replacement article, said closure means being selectively releasable and said receptacle capable of being selectively unwrapped to expose the replacement article, whereupon, following removal from the cavity of the replacement article, at least a part of a user's hand is receivable through said rim and within the cavity and is able to grasp and hold a spent article without touching the spent article; and second closure means adjacent said rim for holding said rim closed after said receptacle has been turned inside out and the spent article enveloped by said second surface, said receptacle and the spent article contained therein thereby being in condition for sanitary disposal.

2. A packaging system as set forth in claim 1 wherein said receptacle is composed of polyethylene.

3. A packaging system as set forth in claim 1 wherein said sealed end of said receptacle includes at least two spaced apart finger elements, each adapted to receive therein at least one digit of the user's hand.

4. A packaging system as set forth in claim 1 wherein said second closure means includes a tie wrap mounted to said receptacle generally coextensive with and adjacent to said rim, said tie wrap adapted to be twisted about itself when said rim is drawn to its substantially closed position to seal said receptacle after receiving therein the spent article.

5. A packaging system as set forth in claim 1 wherein said second closure means includes pressure sensitive tape fixedly applied to said first surface at locations proximate to and generally equidistant from said rim when said rim is drawn to its substantially closed position to hold said rim in its closed position.

6. A packaging system as set forth in claim 1 wherein said second closure means includes a tie wrap received around said first surface with said rim in its closed position at locations proximate to and generally equidistant from said rim when said rim is drawn to its substantially closed condition, said tie wrap being twisted about itself and said first surface.

7. A packaging system as set forth in claim 1 wherein said second closure means includes a pair of tie wraps mounted to said receptacle at spaced apart locations adjacent to said rim, said tie wraps adapted to be twisted about one another when said rim is drawn to its substantially closed position to seal said receptacle after receiving therein the spent article.

8. A packaging system as set forth in claim 1 wherein said first and second closure means are the same structure.

9. A packaging system according to which a protective outer wrap member for a new article also provides for contamination free handling of a spent article and serves as a receptacle for disposal of the spent article comprising: a generally tubular shaped receptacle sealed at one end and having an opening at the other end defined by a rim movable between open and closed positions, said opening being of a size to accept a human hand, said receptacle composed of relatively thin, pliable, liquid impervious material, said receptacle having a first surface defining a cavity extending between said sealed end and said rim and a second surface being the outer surface of said receptacle, said receptacle adapted to receive and protectively envelop a replacement article within the cavity, said rim adapted to be closed to seal the replacement article within the cavity.

10. A packaging system as set forth in claim 9 wherein said first and second closure means are the same structure, said closure means comprising elastic means integral with said receptacle extending continuously therearound adjacent said rim for normally holding said receptacle adjacent said rim in the closed position but capable of being stretched to permit said rim to be moved to the open position.

11. A packaging system as set forth in claim 9 wherein said first and second closure means are the same structure, said receptacle including a substantially continuously extending hem adjacent said rim defining a tube having opposed openings, and said closure means including a draw string having opposed ends extending through said hem, the ends of said draw string extending, respectively, beyond the openings of said tube, said ends capable of being drawn together to move said hem to the closed position and tied to maintain said hem in the closed position, said ends capable of being untied and relaxed to move said hem to the open position.

0 213 241

FIG. 1.

FIG. 2.

FIG. 3.

FIG. 4.

FIG. 5.

FIG. 8.

FIG. 7.

FIG. 9.

FIG. IO.

FIG. II.

FIG. 12.

FIG. 14.

FIG. 13.

FIG. 15.

FIG. 16.

FIG. 17.

FIG. 18.

FIG. 6.

FIG. 19.

FIG. 20.

36

80

30A

21

86
88

82

92
94

90

FIG. 21.

84

82

92

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | FR-A-1 250 934  (A.F. LANE)<br><br>* Page 2, lines 9-19, 36-48; figures 1,2 *<br><br>--- | 1-3,9,11 | A 61 F   13/20 |
| Y | DE-A-3 106 943  (R. PILLICH)<br><br>* Whole document *<br><br>--- | 1-3,9,11 | |
| A,D | US-A-2 750 033  (J.B. PICKENS)<br><br>--- | | |
| A | US-A-3 814 099  (KOBLER)<br><br>----- | | |
| | | | **TECHNICAL FIELDS SEARCHED (Int. Cl.4)**<br><br>A 61 F<br>B 65 D<br>A 41 B |

The present search report has been drawn up for all claims

| Place of search | Date of completion of the search | Examiner |
|---|---|---|
| THE HAGUE | 10-04-1986 | MARTINOZZI G.M.E. |